# EUROPÄISCHE PATENTSCHRIFT

(11) **EP 1 113 755 B1**
(45) Veröffentlichungstag und Bekanntmachung des Hinweises auf die Patenterteilung: **17.12.2003**
(21) Anmeldenummer: 99948780.4
(22) Anmeldetag: 16.09.1999
(51) Int. Cl.: A61B 17/22

(54) **VORRICHTUNG ZUM ERGREIFEN VON KÖRPERN**
DEVICE FOR GRASPING BODIES
DISPOSITIF PERMETTANT DE SAISIR DES CORPS

(30) Priorität: 17.09.1998 DE 19842520
(43) Veröffentlichungstag der Anmeldung: 11.07.2001
(73) Patentinhaber: PFM Produkte für die Medizin Aktiengesellschaft, 50996 Köln (DE)
(72) Erfinder: FREUDENTHAL, Franz, D-52072 Aachen (DE)
(74) Vertreter: Sternagel, Fleischer, Godemeyer & Partner Patentanwälte
(86) Internationale Anmeldenummer: EP9906904
(87) Internationale Veröffentlichungsnummer: WO00016703

(56) Entgegenhaltungen:
- US-A- 5 171 233
- US-A- 5 643 281

## Beschreibung

Die Erfindung betrifft eine Vorrichtung zum Ergreifen von Körpern, Gegenständen oder Partikeln, insbesondere Fremdkörpern, Agglomeraten, Steinen oder anderen organischen Ablagerungen oder Ansammlungen aus menschlichen oder tierischen Gefäßen oder Körperhohlräumen mit einem länglichen proximalen Teilstück und einem distalen, mit einer Schlinge versehenen Teilstück, wobei die Schlinge aus einem flexiblen elastischen Material besteht.

Derartige Vorrichtungen sind bekannt. Sie dienen bei medizinischen Eingriffen beispielsweise als interventionelles Instrument zum Bergen von Fremdkörpern, welche gelegentlich nach Operationen im Körper des Patienten verblieben sind. Es kann nämlich vorkommen, daß bei Verwendung von Kathetern und Führungsdrähten ein distales Ende des Katheters oder Führungsdrahtes versehentlich vom Rest abgetrennt wird und im Körper des Patienten verbleibt. Auch durch Ingestion können Fremdkörper in einen menschlichen oder tierischen Körper gelangen. Besonders schwerwiegend ist es, wenn ein solcher Fremdkörper in einem Körperhohlraum, wie beispielsweise dem Herzen, steckenbleibt. Als Folgen davon kann es zu Septikämien, Lungenembolien, Perforationen und letztlich zu plötzlichem Tod kommen. Weiterhin können mit derartigen Vorrichtungen auch Nierensteine, Gallensteine, Polypen oder andere organische Partikel aus Körperhöhlen, Organen, Organwegen und Gefäßen entfernt werden.

Um derartige Fremdkörper minimal invasiv interventionell aus dem menschlichen oder tierischen Körper zu entfernen, wurden verschiedene Techniken unter Verwendung von Greifmechanismen in Verbindung mit einem Katheter zur Anwendung gebracht. In der deutschen Patentschrift 195 14 534 C2 ist ein Instrument zur Bergung von Fremdkörpern aus menschlichen oder tierischen Gefäßen oder anderen Körperhohlräumen mit einem länglichen proximalen Teilstück und einem distalen Teilstück in Form einer Schlinge beschrieben, wobei die Schlinge aus einem flexiblen superelastischen Material besteht. Das distale Teilstück ist dort bezüglich der Achse, welche durch das längliche proximale Teilstück verläuft, erst zu einer Seite und dann in Form eines geöffneten U's zur gegenüberliegenden Seite gebogen.

Aus der US-Patentschrift 5,171,233 und von J.W. Yedlicka, J.E. Carlson, D.W. Hunter et al. in Radiology 178, 691-693, 1991, wird eine Mikroschlinge beschrieben, welche aus einem länglichen proximalen Teil und einem distalen Teil in Form einer runden oder ovalen Schlaufe aus einem Formgedächtnismaterial besteht. Der distale schlaufenförmige Teil ist im Vergleich zum proximalen Teil abgeknickt und schließt mit diesem einen starren Winkel von beispielsweise 90° ein. Bei dieser Schlinge ist der Fangquerschnitt begrenzt, wodurch ein optimales Ergreifen von Fremdkörpern lediglich dann geschieht, wenn diese in etwa parallel zu dem proximalen Ende und somit parallel zu dem Katheter angeordnet sind, durch den die Schlinge eingeführt wird. Der zum Zuführen der Schlinge verwendete Katheter neigt außerdem dazu, sich seitlich an der Gefäßwand anzulegen. Dies kann sich beim Ausbilden der Schlinge für eine Loop-Bildung sowie dem Einfangvorgang nachteilig auswirken und zu einer größeren Zeitdauer des Einfangens bzw. Bergens des Fremdkörpers bzw. zu verlängerten Durchleuchtungszeiten mit Röntgengeräten führen.

Aus der US 2,756,752 ist eine Vorrichtung zum Enfternen von Fremdobjekten aus Körperhöhlen und insbesondere von Steinen aus dem Ureterbereich bekannt. Die Vorrichtung weist hierzu in Wellenlinienoder Spiralform gebogene Drähte auf, die vorgeformt sind. Diese werden insbesondere von einem Katheter mit länglichem rohrförmigem Körper, der einen mit einer Mehrzahl von wellen- oder wendelförmigen Windungen versehenen Teil aufweist, umgeben. An den wellenlinien- oder spiralförmig gebogenen Drähten - mit diese umgebendem Katheter - bleiben die zu entfernenden Steine hängen, wodurch sie aus der Körperhöhle befreit werden können.

Des weiteren ist aus JP 5-184585(A) eine an ihrem einen Ende mit einer Spirale versehene Vorrichtung zum Ergreifen von Steinen in Körperöffnungen bzw. -gefäßen bekannt. Dabei wird ein Fangelement am frontseitigen Ende der Vorrichtung durch einen Rückhalteteil gehalten und kann dadurch beim Zurückziehen in die Vorrichtung plastisch von der Spiralform in eine langgestreckte Form umgeformt werden.

Die JP 1097446(A) offenbart demgegenüber einen elastischen achtförmig gebogenen Draht mit einer großen Schlinge und einer kleinen Schlinge, die gegeneinander um 180° verwunden sind. Die kleine Schlinge ist durch Drehen des mittleren Teils eines langgestreckten Drahtes um 360° gebildet. Die Enden der großen Schlinge sind an einem Verbindungsstück befestigt. Beim Hineinziehen des elastischen Drahtes in eine flexible Röhre werden die beiden Schlingen zusammengedrückt und langgesteckt. Dabei findet keine plastische Deformation der Schlingen statt.

Der Erfindung liegt die Aufgabe zugrunde, eine Vorrichtung zum Ergreifen von Körpern, Gegenständen oder Partikeln, insbesondere Fremdkörpern, Agglomeraten, Steinen oder anderen organischen Ablagerungen oder Ansammlungen, zu schaffen, mit der ein noch sichereres Einfangen und Bergen von Körpern oder Partikeln jeder Art in den verschiedensten Positionen innerhalb eines körpereigenen Hohlraumes oder Gefäßes mühelos und sicher möglich ist, und bei der insbesondere die geschilderten Nachteile des Standes der Technik vermieden werden.

Die Aufgabe wird durch eine Vorrichtung nach dem Oberbegriff des Anspruchs 1 dadurch gelöst, daß eine zumindest teilweise doppelt gelegte Schlinge mit einem Schlingenzentrum vorgesehen ist, die im Zusammenwirken mit einer länglichen, rohrförmigen, die Vorrichtung zumindest teilweise umgebenden Einrichtung aus dieser primären Konfiguration heraus sekundär konfigurierbar ist, wobei die Schlinge zumindest in ihrer primären Konfiguration von der Längsachse des länglichen proximalen Teilstücks in einem vorbestimmten Winkel abgewinkelt ist. Weiterbildungen der Erfindung sind in den abhängigen Ansprüchen definiert.

Die zumindest teilweise doppelt gelegte Schlinge bildet zwei Schlingenteile, die zueinander so ausgerichtet sind, daß sie eine innere Öffnung und insbesondere diese umgebende Spalten in der primären Konfiguration bilden. Vorzugsweise ist dabei der eine Schlingenteil geschlossen und der andere entweder diesen teilweise umgebend oder diesem teilweise einbeschrieben gebildet. Bei Verändern der primären in die sekundäre Konfiguration geschieht ein Verlagern der inneren Öffnung und/oder Verändern der Form und/oder des Durchmessers der Schlinge bzw. ein Verlagern des Schlingenzentrums der beiden Teile der doppelt gelegten Schlinge.

Dadurch wird eine Vorrichtung zum Ergreifen von Körpern, Gegenständen und Partikeln geschaffen, mittels derer im Vergleich zu der in der DE 195 14 534 C2 geschilderten Vorrichtung eingefangene Körper oder Partikel verschiedenster Abmessungen und Formen aufgrund der doppelten Umschlingung beim Zurückziehen der Schlinge in einen Katheter besser festgehalten werden können. Ein einmal in die doppelt gelegte Schlinge gelangter Körper wird damit durch Zuziehen dieser Schlinge derart fest umgriffen, daß auch bei einer entsprechenden glatten Oberfläche oder einem sehr dünnen Material ein Herausgleiten aus der Schlinge kaum mehr möglich ist. Mittels der erfindungsgemäßen Vorrichtung können aus den verschiedensten Gefäßen und Körperhohlräumen des menschlichen oder tierischen Körpers Körper und Partikel organischen oder nicht organischen Ursprungs entfernt werden. Besonders bevorzugt wird die Vorrichtung bei Eingriffen an Gefäßen, Harn-, Gallen- und Atemwegen sowie dem Magen- und Darmtrakt oder auch bei laparoskopischen Operationen verwendet. Sie kann aber auch zum Ergreifen von Gegenständen wie Drähten oder dergleichen verwendet werden, die in den Körper eines Patienten eingeführt werden sollen.

Als besonders vorteilhaft erweist es sich, daß verschiedene Schlingendurchmesser und Formen mit ein und derselben Schlinge bedarfsgerecht jeweils in Abhängigkeit von der jeweiligen Anwendung erzeugt werden können. Einerseits ist es möglich, die Schlinge in verschiedenen Durchmessern auszubilden, beispielsweise von 10 bis 15 mm in der Kinderheilkunde, von 2 bis 3 mm in der Neurochirurgie, von etwa 5 mm bei der Verwendung mit Coronargefäßen und von 20 bis 40 mm in der Radiologie. Hierbei werden die Drahtdurchmesser der Schlinge vorzugsweise an die entsprechend zu erreichenden Schlingendurchmesser angepaßt. Sie weisen beispielsweise Werte zwischen 0,05 und 0,5 mm auf. Das distale Teilstück kann hierbei beispielsweise im geformten Zustand eine Länge von etwa 25 mm aufweisen.

Zudem können aber auch durch die steuerbar in zahlreichen Variationen veränderbare Form der Schlinge die verschiedensten Behandlungen mit ein und derselben Vorrichtung durchgeführt werden. Wechsel der Form und Größe der Schlinge sind sogar während des Eingriffs möglich. Die Form der Schlinge kann zum einen im wesentlichen rund oder oval mit teilweise doppelt gelegter Schlinge und zum anderen, bei Zusammenwirken mit der länglichen rohrförmigen Einrichtung, vorzugsweise einem Katheter, herzförmig sein.

Aufgrund ihrer besonderen, von außerhalb des Patienten steuerbaren Formgebung weist die erfindungsgemäße Schlinge die Fähigkeit auf, sich an der Wandung eines Gefäßes oder in einem Körperhohlraum selbst zu zentrieren. Der Durchmesser des Gefäßes sollte dabei vorteilhaft kleiner sein als der Durchmesser der Schlinge. Dadurch klemmt sich die Schlinge selbst an der entsprechenden Wandung fest und paßt sich an diese an. Soll ein großer Körper aus z.B. einem Gefäß entfernt werden, ist die Gefäßgröße maßgebend, da die Schlinge auf diese abgestimmt wird. Vorzugsweise wird bei einem größeren Gefäß auch eine größere Schlinge verwendet.

Gerade bei sehr dünnen Materialien, welche als Fremdkörper entfernt werden sollen, erweist sich ein an der Schlinge vorgesehener Vorsprung in Form einer Schlaufe als besonders vorteilhaft, da beim Zuziehen der doppelt gelegten Schlinge der zu entfernende Körper in diese Schlaufe hineingedrängt wird und in dieser festhakt. Besonders bevorzugt ist diese Schlaufe in einem Winkel, insbesondere in einem Winkel von 90°, zu der Ebene der Schlinge angeordnet. Dadurch wird beispielsweise ein flexibler Draht oder ein anderer dünner Fremdkörper bereits so positioniert, daß er ohne größeren Widerstand in den Katheter gezogen werden kann. Im Bereich der Schlaufe wird er beim Hineinziehen in den Katheter entsprechend abgeknickt und hält dadurch besonders gut in diesem Bereich der erfindungsgemäßen Vorrichtung fest.

Anstelle der Schlaufe kann der an der Schlinge vorgesehene Vorsprung auch aus einem aufgefügten Element bestehen oder durch Verschmelzen von zwei Schlingenteilen gebildet sein. Besonders bevorzugt ist das aufgefügte Element buchsenartig oder kugelförmig, weist insbesondere abgerundete Enden auf oder ist eine atraumatische Kugel. Dies hat den Vorteil, daß sowohl Buchse als auch atraumatische Kugel, sofern sie aus einem Material bestehen, welches in einem bildgebenden Gerät, beispielsweise einem Röntgengerät, NMR-Tomographen, etc., sichtbar ist, von außen durch dieses die Bergung des zu entfernenden Körpers beobachtet werden kann.

Die Schlinge kann vorzugsweise auch aus zwei Teilen bestehen, welche im Bereich des Vorsprungs durch Verschmelzen miteinander verbunden sind.

Dies geschieht vorzugsweise alternativ zu dem aufgefügten Element. Selbstverständlich kann ein solches aufgefügtes Element auch auf eine einteilige Schlinge aufgefügt werden, wobei dann vorzugsweise zunächst eine Schlaufe gebildet wird, auf welche eine atraumatische Kugel aufgeschmolzen oder eine entsprechende Buchse aufgesteckt wird.

Bevorzugt ist die Schlinge im wesentlichen senkrecht oder in einem stumpfen Winkel zu der Längsachse des länglichen proximalen Teilstücks der erfindungsgemäßen Vorrichtung angeordnet. Dadurch ist ihre Reichweite sowie der Einzugsbereich zum Erfassen der einzufangenden Körper besonders groß. Vorzugsweise ist der Bereich des distalen Teilstückes zwischen der Schlinge und dem proximalen Teilstück V-förmig ausgebildet. Hierdurch wird eine Art Trichter gebildet, in den die Körper eingefangen werden. Eine weitere Hilfe zum Einfangen der Körper wird durch die Anordnung des Zentrums der Schlinge entweder genau über dem distalen Ende des proximalen Teilstücks oder außerhalb der Längsachse des länglichen proximalen Teilstücks geschaffen. Es werden dadurch in allen Richtungen Spalte, Schlitze oder Überstände der doppelt gelegten Schlinge geschaffen, in welchen sich der einzufangende Körper festhaken kann. Dessen Bergung gestaltet sich dadurch vorteilhaft leichter als beispielsweise bei den bekannten Schlingen, wie sie in der US 5,171,233 oder der DE 195 14 534 C2 geschildert sind. Die Variationsbreite ist bei dem Verlagern des Schlingenzentrums sehr groß und führt zu einem steten Unterschied der primären und der sekundären Konfiguration der Schlinge. Schneidet die Längsachse des proximalen Teilstücks in einer primären Konfiguration nahezu den an der Schlinge gebildeten Vorsprung, faltet sich die Schlinge beim Zurückziehen in die längliche rohrförmige Einrichtung, insbesondere einen Katheter, so zusammen, daß die doppelt gelegte Schlinge in der sekundären Konfiguration einen zu dem Katheter geneigten Bogen und zwei zueinander gedrehte, auf dem Bogen liegende Schlingen bildet. Geht die Längsachse des proximalen Teilstücks in der primären Konfiguration im wesentlichen durch das Zentrum der Schlinge, bildet die Schlinge in einer sekundären Konfiguration eine Herzform aus.

Eine weitere Variationsmöglichkeit besteht auch darin, das Verhältnis zwischen Durchmesser und Form der inneren Schlinge und dem Abstand zum äußeren Teil der Schlinge zu verändern. Die jeweilige Ausbildung der sekundären Konfiguration der Schlinge beim Zurückziehen in einen Katheter oder ähnliches kann dabei ebenfalls anwendungsspezifisch variiert werden.

Besonders bevorzugt besteht die Schlinge aus einem Draht aus Formgedächtnismaterial. Durch Vorsehen des erfindungsgemäßen Vorsprunges kann sie nachfolgend leicht in einen Katheter eingeführt werden. Das gesamte distale Ende der erfindungsgemäßen Vorrichtung wird dabei vollständig gestreckt und nimmt erst bei Verlassen des Katheters innerhalb des Körpers des Patienten vorteilhaft wieder die im Trainingsvorgang eingeprägte Form an. Als Formgedächtnismaterial wird bevorzugt ein sehr elastische Material verwendet, wobei sich beispielsweise eine Titan-Nickel-Legierung, insbesondere Nitinol, eignet, aber auch jeder andere superelastische Werkstoff vorteilhaft verwendet werden kann. Die Schlinge kann zusätzlich mit weiteren Materialien beschichtet sein und/oder werden, die ihr bestimmte gewünschte Eigenschaften verleihen. Hierfür eignen sich besonders Edelmetalle, wie Gold oder Platin. Es können anstelle einer Beschichtung oder zusätzlich zu dieser auch ein oder mehrere Fäden aus Materialien vorgesehen sein, die die Schlinge bei einem bildgebenden Verfahren sichtbar machen, beispielsweise beim Röntgen oder der NMR-Tomographie. Hierfür eigenen sich Materialien wie Gold, Platin, Wolfram oder vergoldetes Wolfram. Selbstverständlich können auch andere, auf dem Gebiet der Chirurgie als geeignet erachtete Werkstoffe verwendet werden, sofern sie eine ausreichende Elastizität und Flexibilität aufweisen.

Besonders bevorzugt sind das distale und das proximale Teilstück der Vorrichtung einstückig, wobei das proximale Teilstück die Enden des für die Schlinge und den V-förmigen Teilbereich verwendeten Drahtes umgibt. Die vorzugsweise im wesentlichen parallel verlaufenden Enden des Drahtes können im proximalen Teilstück der Vorrichtung miteinander verbunden sein, um Verschiebungen der Drahtenden gegeneinander zu vermeiden und eine ausreichende Steifigkeit des proximalen Teilstücks zu bewirken, damit dieses problemlos durch einen Katheter geschoben werden kann. Die Drahtenden können miteinander verklebt oder verdrillt sein. Vorzugsweise werden die beiden Drahtenden des proximalen Teilstücks jeweils mit einer reibungsvermindernden hydrophilen Schicht versehen. Dadurch können die beiden Drahtenden gezielt unabhängig voneinander betätigt werden. Durch Verschieben der Drahtenden gegeneinander kann die Größe der doppelt gelegten Schlinge wie bereits beschrieben verändert werden. Alternativ können die beiden Drahtenden auch fixiert sein, wobei sie am proximalen Ende des proximalen Teilstücks miteinander fest verbunden werden, beispielsweise durch Auffügen einer entsprechenden Buchse oder durch Verschmelzen.

Da bei kleinerem gewünschtem Schlingendurchmesser vorzugsweise auch der Drahtquerschnitt verändert wird, kann dies durch Vorsehen eines Drahtes mit einer oder mehrerer Drahtseelen und/oder einer oder mehrerer Drahtlitzen geschehen. Die Drahtlitze kann an einigen Stellen dichter oder dicker auf die Drahtseelen gewickelt sein, wodurch partiell unterschiedliche Drahtquerschnitte entstehen. Derselbe Effekt läßt sich durch Verändern der Anzahl der Drahtseelen und/oder der Drahtlitzen erzeugen. Vorteilhaft kann durch die Verwendung von Drahtlitzen darüber hinaus die Knickstabilität des Drahtes der Schlinge erhöht werden. Bei einem auftretenden Bruch einer einzelnen Drahtfaser wird vorteilhaft die Funktionsfähigkeit des Drahtes als solchem noch nicht beeinträchtigt.

Besonders bevorzugt weist der Draht einen im wesentlichen runden, ovalen, rechteckigen oder mehreckigen Querschnitt auf, wobei er vorzugsweise als Flachdraht vorgesehen ist. Die Auswahl der jeweils geeigneten Querschnittsform ist von der Anwendung abhängig. Beispielsweise kann eine für die Polypenentfernung benötigte Schnittebene besonders vorteilhaft durch einen eckigen, insbesondere rechteckigen Querschnitt bereitgestellt werden. Insbesondere kann auch ein sternförmiger Querschnitt verwendet werden, wodurch die Haltekraft der Schlinge erhöht wird, da die herausragenden Kanten des sternförmigen Querschnitts besonders gut in einen weichen organischen zu entfernenden Körper einschneiden und diesen dadurch zusätzlich festhalten können.

Vorzugsweise besteht das proximale Teilstück aus einem Material, welches über Flexibilität in Verbindung mit Steifigkeit und ausreichender Knickstabilität verfügt. Selbstverständlich kann das proximale Teilstück aber auch aus dem gleichen Material bestehen wie das distale Teilstück, wobei aus Kostenersparnisgründen die Verwendung eines kostengünstigeren Materials mit den genannten Eigenschaften für das proximale Teilstück bevorzugt wird. Zusätzlich kann das proximale Teilstück auch mit einer Spirale umgeben sein und/oder mit einer Ummantelung, welche reibungsreduzierend wirkt. Eine Ummantelung kann z.B. als hydrophile Gleitschicht ausgebildet sein. Dadurch wird das Verschieben innerhalb des Katheters, durch den die Vorrichtung in den Körper des Patienten eingeführt wird, erleichtert.

Besonders bevorzugt kann das proximale Teilstück am proximalen Ende mit einem Torquer versehen sein, der aufgeschraubt die Drahtenden des distalen Teilstücks fest umgreift. Mit Hilfe des Torquers kann die Vorrichtung innerhalb eines Katheters leicht gedreht und verschoben werden.

Zur näheren Erläuterung der Erfindung werden im folgenden Ausführungsbeispiele anhand der Zeichnungen beschrieben. Diese zeigen in:
- Fig. 1: eine perspektivische Ansicht einer ersten Ausführungsform einer erfindungsgemäßen Vorrichtung zum Ergreifen von Fremdkörpern,
- Fig. 2: eine Rückansicht der erfindungsgemäßen Vorrichtung gemäß Fig. 1,
- Fig. 3: eine Frontansicht der Vorrichtung gemäß Fig. 1,
- Fig. 4: eine Draufsicht auf die Vorrichtung gemäß Fig. 1,
- Fig. 5: eine Draufsicht auf eine zweite Ausführungsform der erfindungsgemäßen Vorrichtung,
- Fig. 6: eine Draufsicht auf eine dritte Ausführungsform der erfindungsgemäßen Vorrichtung,
- Fig. 7: eine Prinzipskizze der einzelnen Schritte des Auseinanderfaltens einer erfindungsgemäßen Vorrichtung aus einem Katheter heraus, durch den sie einen Patienten eingeführt werden kann,
- Fig. 8: eine perspektivische Ansicht einer weiteren Ausführungsform der erfindungsgemäßen Vorrichtung während eines Bergungsvorganges mit Fremdkörper,
- Fig. 9: eine perspektivische Ansicht der Vorrichtung gemäß Fig. 8 während des Zurückziehens in den Katheter beim Bergen des Fremdkörpers,
- Fig. 10: eine Draufsicht auf eine erfindungsgemäße Schlinge in sekundärer Konfiguration,
- Fig. 11: eine Frontansicht einer anderen sekundären Konfiguration der Schlinge gemäß Fig. 10, und
- Fig. 12: eine Draufsicht und eine entsprechende Seitenansicht einer weiteren Ausführungsform einer erfindungsgemäßen Schlinge.

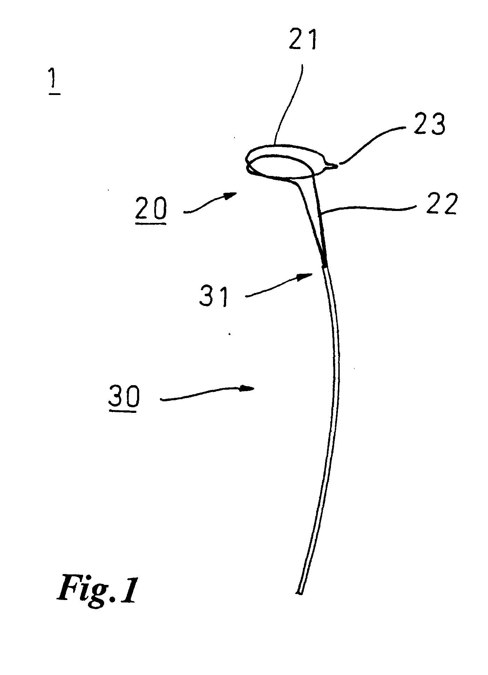

In Fig. 1 ist eine perspektivische Ansicht einer ersten Ausführungsform einer Vorrichtung 1 zum Ergreifen eines Körpers oder Partikels dargestellt. Die Vorrichtung weist ein distales Teilstück 20 und ein proximales Teilstück 30 auf. Das proximale Teilstück 30 ist länglich und ist an seinem distalen Ende 31 mit dem distalen Teilstück 20 versehen. Das distale Teilstück 20 weist eine doppelt gelegte Schlinge auf. Die doppelt gelegte Schlinge ist gegenüber der gedachten verlängerten Längsachse des länglichen proximalen Teilstücks abgewinkelt, insbesondere in einem stumpfen Winkel oder in einem Winkel von etwa 90°. Falls der spezielle Anwendungsfall dies erfordert, können auch spitze Winkel vorgesehen sein.

Die doppelt gelegte Schlinge ist aus einem Draht gebildet, welcher sich V-förmig von dem distalen Ende 31 des proximalen Teilstücks 30 heraus erstreckt. An den V-förmigen Teilbereich 22 schließen sich die Windungen der doppelt gelegten Schlinge 21 über den bereits genannten Winkel an.

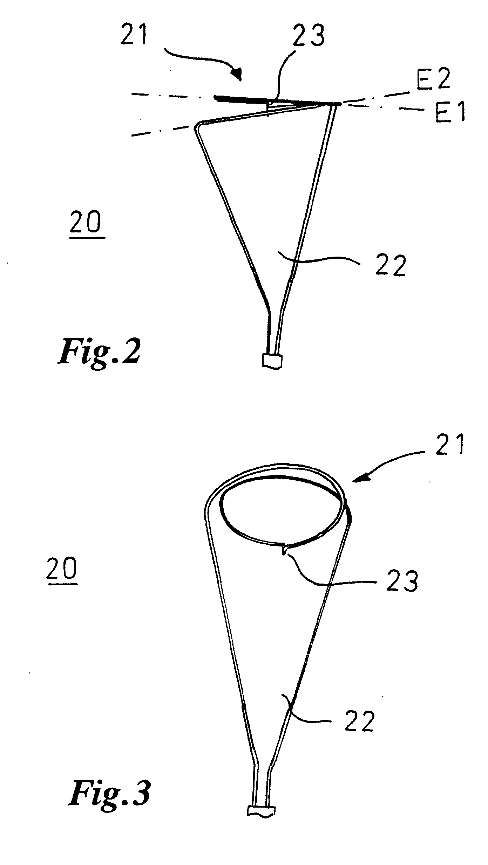

Die Schlinge 21 weist einen Vorsprung 23 auf. Der Vorsprung 23 ist in Form einer Schlaufe vorgesehen, welche im vorderen Bereich an die Schlinge angeformt ist. Bevorzugt ist diese Schlaufe um 90° gedreht zu der Ebene der Schlinge 21 gebildet. Dies kann besser den Figuren 2 und 3 entnommen werden.

Fig. 2 zeigt hierbei eine Rückansicht des distalen Teilstücks 20 der Vorrichtung 1 gemäß Fig. 1. Aus dieser Ansicht wird deutlich, daß die doppelt gelegte Schlinge 21 aus zwei Teilen aufgebaut ist, die zueinander versetzt in sich schneidenden Ebenen liegen. Dies ist durch die gestrichelten Linien in Fig. 2 angedeutet, wobei E1 und E2 die beiden Ebenen angeben. Eine Querverbindung zwischen diesen beiden Ebenen findet durch den Vorsprung 23 statt, welcher hinsichtlich seiner Anordnungsebene die beiden anderen Ebenen durchschneidet. Dies kann noch besser Fig. 3 entnommen werden, die eine Frontansicht der Vorrichtung 1 gemäß Fig. 1 und 2 darstellt. Der zwischen den Ebenen E1 und E2 gebildete Winkel ist frei wählbar und kann beispielsweise auch im wesentlichen Null sein. Vorzugsweise wird der Winkel zwischen diesen beiden Ebenen so gewählt, daß eine weitere Möglichkeit zum Einfangen des Fremdkörpers geschaffen wird. Dieser kann dann auch eingefangen werden, wenn er nicht direkt in die geschlossene Öffnung der Schlinge oder den V-förmigen Teilbereich gerät. Beim Zurückziehen des distalen Teilstücks 20 in einen Katheter wird ein zwischen die beiden Ebenen E1 und E2 geratener Fremdkörper festgehalten und kann nachfolgend entweder weiter eingefädelt werden oder zumindest so weit positioniert werden, daß er in die Schlinge selbst eingefädelt werden kann.

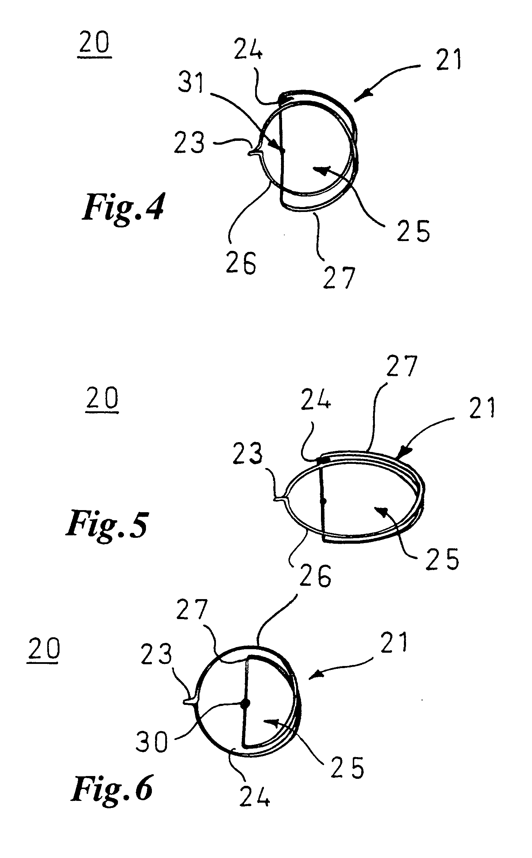

Die Schlinge als solche zeigt Fig. 4 in der Draufsicht. Hierbei ist deutlich zu erkennen, daß aufgrund der doppelt gelegten Schlinge noch weitere Spalten 24 geschaffen werden, in welchen ein Fremdkörper sich verfangen kann. Bei bestimmten Anwendungen können die Spalten 24 sehr viel größer sein als dargestellt, wobei das Verhältnis der Spalten zu einer inneren Öffnung 25 sich dabei ändert.

Die doppelt gelegte Schlinge 21 kann so gegenüber dem distalen Ende 31 des proximalen Teilstücks, welches lediglich in der Draufsicht als Kreis zu erkennen ist, angeordnet sein, so daß eine große Öffnung 25 geschaffen wird, in welcher auch größere Fremdkörper sehr gut eingefangen werden können. Ein sicherer Halt ist auch beim Zurückziehen der Vorrichtung in einen Katheter durch die doppelte Umschlingung gegeben, da sich die Größe der Öffnung dabei verringert. In der Draufsicht ist also das distale Ende 31 außermittig von der doppelt gelegten Schlinge 21 vorgesehen. In anderen Anwendungsfällen kann es jedoch auch so sein, daß eine zentrale Anordnung (in der Draufsicht) des distalen Endes 31 in der doppelt gelegten Schlinge 21 wünschenswert ist, wie in Fig.5 gezeigt. Die gewünschte primäre Form der Schlinge wird deren Material eingeprägt. Vorzugsweise wird daher ein Formgedächtnismaterial verwendet.

Der Vorsprung 23 kann entweder als Schlaufe gebildet werden, wobei dann die Schlinge 21 aus einem vollständig gebogenen Teil besteht. Der Vorsprung kann aber auch als Nahtstelle zwischen zwei Hälften dieser Schlinge gebildet werden. In diesem Fall kann entweder ein Verschmelzen dieser Nahtstelle oder aber ein Auffügen eines separaten Elementes erfolgen. Als separates Element eignet sich insbesondere eine atraumatische Kugel, welche zum einen das Einführen in einen Katheter erleichtert und zum anderen Verletzungen des Patienten verhindert. Ebenso kann aber auch eine andere Buchse auf eine solche Nahtstelle aufgefügt werden.

Neben der in Fig. 4 dargestellten Form kann die Schlinge aber auch beispielsweise oval sein, wie in Fig. 5 in der Draufsicht dargestellt. Hierbei ist der Bereich der Öffnung 25 entsprechend länglicher geformt, was zwecks Anpassung an bestimmte zu entfernende Körper sich als vorteilhaft erweisen kann. Eine andere Variante besteht darin, den Teil 26 der Schlinge 21, welcher im wesentlichen geschlossen ist, anders als in Fig. 4 und 5 gezeigt, nach außen zu verlegen, wobei der äußere Schlingenteil 27 dann zu einem inneren wird, wie in Fig. 6 zu sehen. Auch dabei ist die Form der im wesentlichen geschlossenen Schlinge vielfältig variierbar und kann beispielsweise auch oval sein. Darüber hinaus sind auch Mischformen möglich, bei denen lediglich eine Hälfte des äußeren Schlingenteils 27 außerhalb des im wesentlichen geschlossenen Schlingenteils 26 liegt, wohingegen der andere Teil innerhalb von diesem liegt.

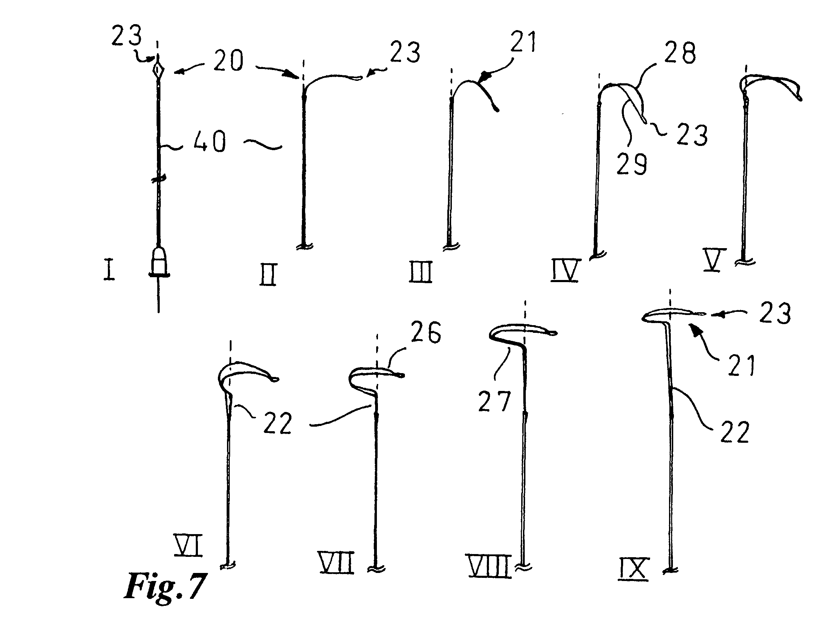

In Fig. 7 sind neun Ansichten eines sich entfaltenden distalen Teilstücks 20 der erfindungsgemäßen Vorrichtung dargestellt. Im ersten Schritt I wird zunächst aus einem Katheter 40 der vordere Teil der Schlinge 21, nämlich der Vorsprung 23 herausgeschoben. Hierbei ist bereits zu erkennen, daß Vorsprung und Schlinge gegeneinander vertwistet sind. Im Schritt II, der Seitenansicht der Ansicht I, ragt bereits ein größerer Teil der Schlinge aus dem Katheter 40 heraus. Zwischen Schlinge und Katheter ist ein Winkel von etwas mehr als 90° ausgebildet. Im Schritt III biegt sich der sich später zum geschlossenen Teil 26 ausbildende Teil der Schlinge 21 nach unten zu dem Katheter hin, wobei die beiden Hälften der Schlinge im wesentlichen noch parallel zueinander liegen. Im Schritt IV ändert sich dies, wobei sich die beiden Hälften 28, 29 der Schlinge bereits auseinander bewegen unter Beibehalten der Wölbung zum Katheter hin.

Im Schritt V reckt sich die Schlinge von dem Katheter nach oben, wobei eine Wölbung verbleibt und die Ausbildung der geschlossenen Schlinge 26 fortschreitet. Im Schritt VI bildet sich ein hakenförmiger Ansatz aus durch den Übergang in den V-förmigen Teilbereich 22. Die Wölbung der Schlinge ist jedoch weiterhin noch verhältnismäßig ausgeprägt. Sie nimmt erst im Schritt VII weiter ab, bei dem ein größerer Teil des V-förmigen Teilbereiches 22 aus dem Katheter herausgeschoben ist. Die durch den Katheter vorgegebene Achse (gestrichelt dargestellt) liegt in diesem Schritt im wesentlichen im Zentrum des sich ausbildenden, im wesentlichen geschlossenen Schlingenteils 26. Durch das weiter fortgeführte Herausschieben des V-förmigen Teilbereichs 22 nähert sich die Schlinge diesem Teilbereich 22 weiter an, wodurch der Haken eine flachere Form bekommt und sich das äußere Schlingenteil 27 ausbildet, wie im Schritt VIII gezeigt.

Der letzte dargestellte Schritt IX zeigt, daß sich die Schlinge 21 und der V-förmige Teilbereich 22 immer weiter annähern, wobei sich der V-förmige Teilbereich leicht zu der Längsachse des Katheters neigt. In Abhängigkeit von der gewünschten Form des distalen Teilstücks 20 wird dabei auch der im wesentl i chen geschlossene Teil 26 der Schlinge 21 so weit verschoben, daß die Öffnung 25, wie sie in den Fig. 4 bis 6 gezeigt ist, ausgebildet werden kann.

Durch die verschiedenen Formen und Schlingendurchmesser, welche sich beim Auseinanderfalten des distalen Teilstücks 20 ausbilden können, ist die Möglichkeit gegeben, die erfindungsgemäße Vorrichtung lediglich so weit aus dem Katheter herauszuschieben, wie es für den Anwendungsfall erforderlich ist. Hierbei können also die Form und der Durchmesser des distalen Teilstücks angepaßt ausgewählt werden.

Wird eine Schlinge verwendet, deren Vorsprung 23 in der Nähe der Längsachse des proximalen Teilstücks liegt, gestalten sich die Schritte VI bis IX anders als in Fig. 7 dargestellt. Die Krümmung der Schlinge zu dem Katheter 40 hin nimmt noch weiter als im Schritt V gezeigt zu. In der Draufsicht wirken die Schlingenteile 26, 27 als seien zwei geschlossene Schlingen verdreht zueinander, miteinander in diesem Übergangsbereich verbunden übereinander angeordnet. Der Vorsprung 23 schiebt sich nachfolgend parallel zu der Katheterwand bis zu der oberen Öffnung des V-förmigen Teilbereichs 22. Beim weiteren Herausschieben der Vorrichtung aus dem Katheter 40 schwenkt der Schlingenteil 26 in den Teil 27 hinein und der V-förmige Teilbereich öffnet sich so weit wie ihm als primäre Konfiguration eingeprägt wurde.

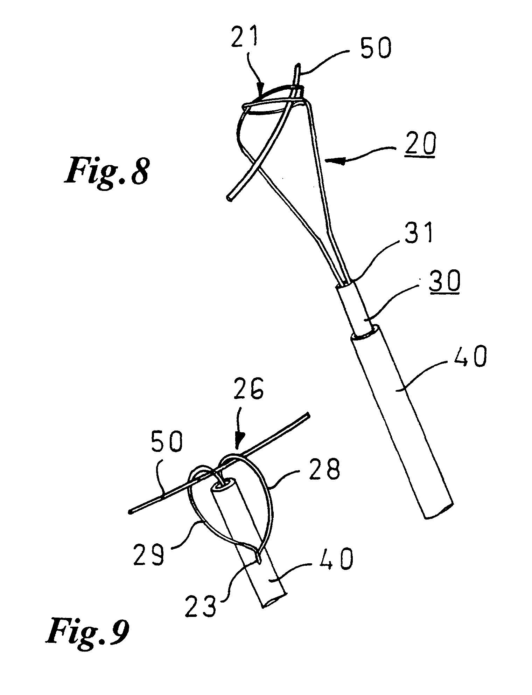

Der Bergungsvorgang als solcher ist in den Fig. 8 und 9 dargestellt. Beide zeigen perspektivische Ansichten des Katheters 40 mit in diesem angeordneten proximalen Teilstücken 30, dessen distales Ende 31 sowie das distale Teilstück 20 in vollständig ausgefahrener Position (Fig. 8) bzw. das distale Teilstück 20 in teilweise eingezogener Position (Fig. 9). Von dem distalen Teilstück 20 ist dabei ein Fremdkörper 50 ergriffen, der sich in Fig. 8 innerhalb der doppelt gelegten Schlinge 21 befindet.

Wird das distale Teilstück 20 zusammen mit dem proximalen Teilstück 30 in den Katheter 40 hineingezogen, wird der Fremdkörper 50 in dem Übergangsbereich von dem im wesentlichen geschlossenen Teil 26 zu dem äußeren Schlingenteil 27 eingeklemmt und festgehalten (Fig. 9). Ein Verlieren des eingefangenen Fremdkörpers 50 beim Zurückführen der Vorrichtung in den Katheter kann dadurch vorteilhaft vermieden werden.

Um einen solchen Fremdkörper 50 zu bergen, wird zunächst ein vorzugsweise reibungsarmer Katheter 40 operativ auf bekannte Weise in den Körper des Patienten bis an die gewünschte Stelle eingeführt. Die erfindungsgemäße Vorrichtung wird, sofern sie sich noch nicht innerhalb des Katheters befindet, wenn sich das distale Ende des Katheters neben dem Fremdkörper befindet, mit dem Vorsprung 23 zuerst in das andere, proximale, außerhalb des Patienten befindliche Ende des Katheters eingeführt. Der Vorsprung 23 kann dabei als Einführhilfe dienen. Es kann aber auch eine zusätzliche Einführhilfe, z.B. in Form eines kleinen Röhrchens verwendet werden.

Die innerhalb des Katheters langgestreckte Schlinge 21 entfaltet sich am Ende des Katheters vollständig wieder, wie in Fig. 7 dargestellt. Wie bereits erwähnt, kann direkt während des Eingriffs vor Ort entschieden werden, wie weit, d.h. bis zu welcher der zahlreichen sekundären Konfigurationen oder Schritte, die Schlinge aus dem Katheter herausgeführt werden soll, um den Fremdkörper optimal einfangen zu können. Das Verschieben der Vorrichtung innerhalb des Katheters kann vorzugsweise mittels eines Torquers geschehen, der vorteilhaft am proximalen Ende des proximalen Teilstücks 30 angebracht wird.

Ist der Fremdkörper 50 von dem distalen Teilstück 20 erfaßt, wird dieses, wie in den Fig. 8 und 9 dargestellt, wieder in den Katheter zurückgezogen. Der Festkörper verklemmt sich (siehe Fig. 9) und wird anschließend mit oder ohne Katheter aus dem Körper des Patienten entfernt. Insbesondere bei größeren Körpern, insbesondere Fremdkörpern, kann hierzu ein zusätzlicher interventioneller oder operativer Eingriff erforderlich sein. Soll ein flexibler Fremdkörper geborgen werden, kann dieser beispielsweise auch durch den Katheter hindurch entfernt werden. Nachfolgend wird dann der Katheter wieder aus dem Körper des Patienten herausgezogen.

Die erfindungsgemäße Vorrichtung kann auch zur Loop-Bildung verwendet werden. Dabei wird durch ein Loch zwischen arteriellem und venösem System ein sog. Loop mittels eines Drahtes gebaut. Der Draht wird durch eine Arterie in den Körper eines Patienten eingeführt, geht völlig durch den Körper des Patienten hindurch, durch das Loch zwischen den Systemen und durch eine Vene des Patienten wieder heraus. Zum Durchführen des Drahtes durch die Blutsysteme des Körpers des Patienten wird die erfindungsgemäße Vorrichtung außerhalb des Körpers des Patienten mit dem Draht versehen (Fig.9), in eine Arterie eingeführt, durch den Körper, das Loch zwischen den Systemen und eine Vene wieder aus dem Körper des Patienten heraus geschoben. Die erfindungsgemäße Vorrichtung erweist sich also auch hierbei als vorteilhaft, da mit ihr ein beliebig geformter Körper oder Gegenstand sicher ergriffen und bewegt werden kann.

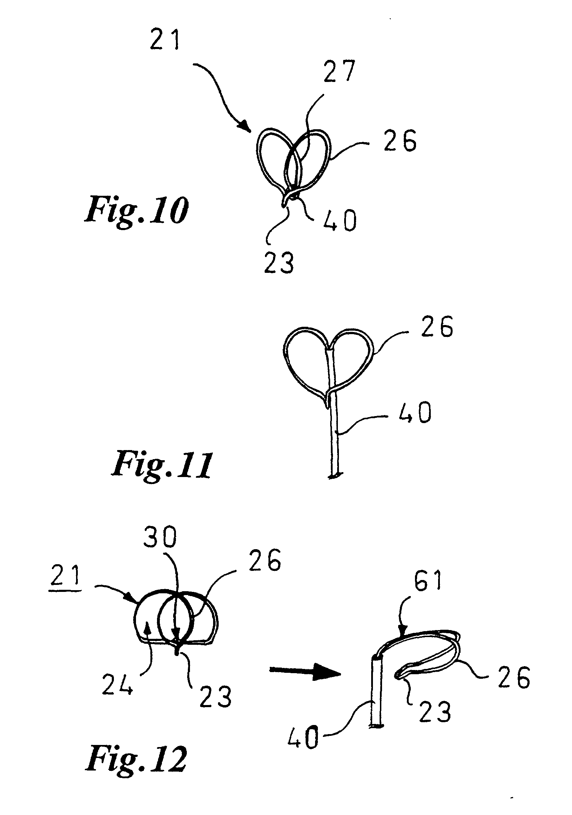

In Fig. 10 und 11 sind eine Draufsichten auf sekundäre Konfigurationen einer Schlinge 21 dargestellt, deren primäre Konfiguration der in Fig. 4 gezeigten entsprechen kann. Beim Zurückziehen der Schlinge in den Katheter 40 nimmt die Schlinge eine Herzform an. Fig. 10 zeigt dabei ein Vorstufe von Fig. 11, wobei in Fig. 11 die Schlinge bereits umgeklappt ist und sich an die Katheterwandung angelegt hat. Aufgrund der Spannung, die dabei von der Schlinge ausgeübt wird, kann gerade auch in dieser Konfiguration die Schlinge zur Einfangen verwendet werden. Der Durchmesser der Schlinge kann bei der Ausbildung der Herzform beispielsweise 15 mm betragen, wohingegen er bei der primären Form (Siehe Fig. 4 bis 6) z.B. lediglich 10 mm betragen hat.

Fig. 12 zeigt eine Draufsicht auf eine weitere Ausführungsform einer erfindungsgemäßen Schlinge 21. Der innere Schlingenteil 26 ist im Verhältnis zu den Spalten 24 klein ausgebildet. Der Vorsprung 23 ist im wesentlichen direkt auf der Längsachse durch das proximale Teilstück 30 angeordnet, vgl. auch Fig. 7, Schritt IX. In der zweiten Darstellung in Fig.12 ist eine perspektivische Ansicht einer sekundären Konfiguration dieser Schlinge gezeigt. Der Auffaltungszustand entspricht etwa dem in Fig. 7, Schritt VI dargestellt. Der Vorsprung 23 zeigt auf den Katheter 40, und der sich ausbildende Schlingenteil 26 ist sehr stark gekrümmt, wobei zudem der Übergangsbereich 61 zu dem noch innerhalb des Katheters befindlichen Schlingenteil 27 zu erkennen ist. Wo eine solche Hakenform zum Entfernen eines Körpers oder Partikels erwünscht ist, kann sie mühelos aus der völlig anders geformten primären Konfiguration der Schlinge gebildet werden. Der Vorgang und die gewünschte Form können auf einem entsprechenden bildgebenden Gerät verfolgt werden.

Neben den in den verschiedenen Figuren dargestellten Ausführungsformen können auch zahlreiche andere Varianten anwendungsspezifisch ausgestaltet werden, welche ebenfalls eine erfindungsgemäß ausgestaltete doppelt gelegte Schlinge aufweisen. Der Querschnitt des für die Schlinge verwendeten Drahtes kann anwendungsspezifisch variiert werden, insbesondere rund, oval, rechteckig, mehreckig und/oder flach sein. Auch hier wird anwendungsspezifisch eine geeignete Querschnittsform ausgewählt.

### Bezugszeichenliste

- 1: Vorrichtung zum Ergreifen

- 20: distales Teilstück
- 21: doppelt gelegte Schlinge
- 22: V-förmiger Teilbereich
- 23: Vorsprung
- 24: Spalte
- 25: Öffnung
- 26: im wesentlichen geschlossener Teil
- 27: äußerer Schlingenteil
- 28: Hälfte
- 29: Hälfte

- 30: proximales Teilstück
- 31: distales Ende

- 40: Katheter

- 50: Fremdkörper

- 61: Übergangsbereich

- E1: Ebene 1
- E2: Ebene 2

## Patentansprüche

1. Vorrichtung zum Ergreifen von Körpern, Gegenständen oder Partikeln, insbesondere Fremdkörpern, Agglomeraten, Steinen oder anderen organischen Ablagerungen oder Ansammlungen aus menschlichen oder tierischen Gefäßen oder Körperhohlräumen, mit einem länglichen proximalen Teilstück (30) und einem distalen, mit einer Schlinge versehenen Teilstück (20), wobei die Schlinge aus einem flexiblen elastischen Material besteht und in eine längliche, rohrförmige, die Vorrichtung zumindest teilweise umgebende Einrichtung (40) hineinziehbar und aus dieser herausschiebbar ist, wobei die Ebene der Schlinge zumindest in ihrer eingeprägten primären Konfiguration von der Längsachse des länglichen proximalen Teilstücks (30) in einem Winkel abgewinkelt ist,
**dadurch gekennzeichnet, daß**
eine zumindest teilweise doppelt gelegte Schlinge (21) mit einem Schlingenzentrum vorgesehen ist, die beim Hineinziehen in und Herausschieben aus der Einrichtung (40) aus einer eingeprägten primären Konfiguration hinsichtlich ihres Schlingenzentrums und/oder ihrer Form und/oder ihres Durchmessers veränderbar ist .

2. Vorrichtung nach Anspruch 1,
**dadurch gekennzeichnet, daß**
ein V-förmiger Teilbereich (22) vorgesehen ist, der sich zwischen der Schlinge (21) und dem proximalen Teilstück (30) erstreckt.

3. Vorrichtung nach Anspruch 1 oder 2,
**dadurch gekennzeichnet, daß**
der Schlingendurchmesser und/oder die Form der Schlinge bei der Verwendung der Vorrichtung (1) mit einer rohrförmigen, länglichen, die Vorrichtung zumindest teilweise umgebenden Einrichtung (40) steuerbar veränderbar ist.

4. Vorrichtung nach Anspruch 1, 2 oder 3,
**dadurch gekennzeichnet, daß**
das Zentrum der Schlinge (21) im wesentlichen außerhalb, insbesondere direkt über der Längsachse des länglichen proximalen Teilstücks (30) positioniert ist.

5. Vorrichtung nach Anspruch 1, 2, 3 oder 4,
**dadurch gekennzeichnet, daß**
die Schlinge (21) in der primären Konfiguration eine im wesentlichen runde oder ovale und in einer sekundären Konfiguration eine Herzform annimmt.

6. Vorrichtung nach einem der vorstehenden Ansprüche,
**dadurch gekennzeichnet, daß**
die Formgebung der Schlinge (21) so einstellbar ist, dass die Schlinge sich innerhalb des Körperhohlraumes oder Gefäßes selbst zentriert und sich an die Wandung des Gefäßes oder Hohlraumes anpaßt.

7. Vorrichtung nach einem der vorstehenden Ansprüche,
**dadurch gekennzeichnet, daß**
die Ebene der Schlinge (21) zu der Längsachse des länglichen proximalen Teilstücks (30) und dem V-förmigen Teilbereich (22) in einem Winkel, insbesondere im wesentlichen senkrecht, angeordnet oder die Schlinge (21) zu der Längsachse des länglichen proximalen Teilstücks (30) und dem V-förmigen Teilbereich (22) und/oder zu der rohrförmigen, länglichen Einrichtung (40) hin in einem Bogen gekrümmt oder gewölbt ist.

8. Vorrichtung nach einem der vorstehenden Ansprüche,
**dadurch gekennzeichnet, daß**
die doppelt gelegte Schlinge zwei Hälften (28, 29) aufweist, die zueinander versetzt in sich schneidenden Ebenen (E1, E2) liegen.

9. Vorrichtung nach Anspruch 8,
**dadurch gekennzeichnet, daß**
die beiden Hälften einteilig miteinander oder aus zwei Teilen zusammengefügt sind, insbesondere die beiden Hälften eine Verbindungsstelle aufweisen, die einen Vorsprung (23) bildet.

10. Vorrichtung nach Anspruch 9,
**dadurch gekennzeichnet, daß**
der Vorsprung (23) schlaufenförmig ist oder aus einem aufgefügten Element besteht oder durch Verschmelzen von zwei Schlingenteilen (28, 29) gebildet ist.

11. Vorrichtung nach Anspruch 10,
**dadurch gekennzeichnet, daß**
das aufgefügte Element buchsenartig oder kugelförmig, insbesondere eine atraumatische Kugel ist.

12. Vorrichtung nach einem der Ansprüche 9 bis 11,
**dadurch gekennzeichnet, daß**
der schlaufenförmige Vorsprung (23) in einem Winkel zu der oder den Ebenen (E1, E2) der Schlinge steht und/oder im wesentlichen parallel zu der Längsachse des proximalen länglichen Teilstücks (30) angeordnet ist.

13. Vorrichtung nach einem der vorstehenden Ansprüche,
**dadurch gekennzeichnet, daß**
das distale Teilstück zwischen der doppelt gelegten Schlinge und dem länglichen proximalen Teilstück einen V-förmigen Teilbereich (22) aufweist.

14. Vorrichtung nach einem der vorstehenden Ansprüche,
**dadurch gekennzeichnet, daß**
distale und proximale Teilstücke der Vorrichtung einstückig sind, wobei das proximale Teilstück (30) die Enden des für die Schlinge (21) und den V-förmige Teilbereich (22) verwendeten Drahtes umfaßt.

15. Vorrichtung nach einem der vorstehenden Ansprüche,
**dadurch gekennzeichnet, daß**
die Schlinge einen Draht aufweist, der eine oder mehrere Drahtlitzen aufweist, insbesondere der Schlingendraht eine oder mehrere Drahtseelen, umwickelt von einer oder mehreren Drahtlitzen aufweist.

16. Vorrichtung nach einem der vorstehenden Ansprüche,
**dadurch gekennzeichnet, daß**
der Draht einen im wesentlichen runden, ovalen, rechteckigen, mehreckigen, sternförmigen und/oder flachen Querschnitt aufweist und der Durchmesser des Drahtes über die Drahtlänge im wesentlichen konstant oder anwendungsspezifisch variiert ist.

17. Vorrichtung nach einem der vorstehenden Ansprüche,
**dadurch gekennzeichnet, daß**
die Schlinge aus einem Draht aus Formgedächtnismaterial, insbesondere aus einem superelastischen Material besteht.

18. Vorrichtung nach einem der vorstehenden Ansprüche,
**dadurch gekennzeichnet, daß**
die Schlinge mit einem Edelmetall beschichtet ist und/oder einen oder mehrere Fäden oder eine Beschichtung aus einem unter einem bildgebenden Gerät sichtbaren Material aufweist.

## Claims

1. An instrument for grasping bodies, articles or particles, in particular foreign bodies, agglomerates, stones or other organic deposits or accumulations from human or animal vessels or body cavities, having an elongate proximal section (30) and a distal section (20) which is provided with a noose, the noose consisting of a flexible elastic material, and is drawable in and pushable out of an elongate, tubular device (40) which at least partly envelops the instrument, where the noose is angled at least in its initial configuration at a predetermined angle from the long axis of the elongate proximal section (30), wherein an at least partly two-ply noose (21) with a noose center is provided which can, be converted from an initial impressed configuration with regard to the noose center and/or shape and/or diameter by drawing in and pushing out of the device (40)..

2. The instrument as claimed in claim 1, wherein a V-shaped region (22) is provided, which extends between the noose (21) and the proximal section (30).

3. The instrument as claimed in claim 1 or 2, wherein the noose diameter and/or the shape of the noose can be changed controllably on use of the instrument (1) using a tubular, elongate device (40) which at least partly envelops the instrument.

4. The instrument as claimed in claim 1, 2 or3, wherein the center of the noose (21) is positioned essentially outside, especially directly over, the long axis of the elongate proximal section (30).

5. The instrument as claimed in claim 1, 2, 3 or4, wherein the noose (21) in the initial configuration assumes an essentially circular or oval shape and in a second configuration a heart shape.

6. The instrument as claimed in any of the preceding claims, wherein the shaping of noose (21) is adjustable such that the noose is self-centering inside the body cavity or vessel and adapts itself to the wall of the vessel or cavity.

7. The instrument as claimed in any of the preceding claims, wherein the noose (21) plane is disposed at an angle, especially essentially perpendicular to the long axis of the elongate proximal section (30) and the V-shaped region (22) or the noose (21) is curved in an arch or has a convexity toward the long axis of the elongate proximal section (30) and the V-shaped region (22) and/or toward the tubular, elongate device (40).

8. The instrument as claimed in any of the preceding claims, wherein the two-ply noose has two halves (28, 29) which lie in intersecting planes (E1, E2) displaced with respect to one another.

9. The instrument as claimed in claim8, wherein the two halves together are in one piece or are connected together from two parts, in particular the two halves have a connection point which forms a projection (23).

10. The instrument as claimed in claim9, wherein the projection (23) is loop-shaped or consists of an attached element or is formed by fusing two noose parts (28, 29).

11. The instrument as claimed in claim 10, wherein the attached element is sleeve-like or spherical, in particular is an atraumatic sphere.

12. The instrument as claimed in any of claims 9 toll, wherein the loop-shaped projection (23) is at an angle to the plane(s) (E1, E2) of the noose and/or is disposed essentially parallel to the long axis of the proximal elongate section (30).

13. The instrument as claimed in any of the preceding claims, wherein the distal section has a V-shaped region (22) between the two-ply noose and the elongate proximal section.

14. The instrument as claimed in any of the preceding claims, wherein distal and proximal sections of the instrument are in one piece, with the proximal section (30) encircling the ends of the wire used for the noose (21) and the V-shaped region (22).

15. The instrument as claimed in any of the preceding claims, wherein the noose has a wire which has one or more wire strands, especially the noose wire has one or more cores, wrapped by one or more wire strands.

16. The instrument as claimed in any of the preceding claims, wherein the wire has an essentially circular, oval, rectangular, polygonal, starshaped and/or flat cross section, and the diameter of the wire varies essentially continuously or for the specific use along the length of the wire.

17. The instrument as claimed in any of the preceding claims, wherein the noose consists of a wire of shape memory alloy, in particular of a superelastic material.

18. The instrument as claimed in any of the preceding claims, wherein the noose is coated with a noble metal, and/or has one or more threads or a coating of a material which is visible under an imaging apparatus.

## Revendications

1. Dispositif pour saisir des corps, des objets ou des particules, notamment des corps étrangers, agglomérats, calculs ou autres dépôts ou accumulations organiques se trouvant dans des vaisseaux ou cavités corporelles chez les humains ou les animaux, comprenant une pièce partielle proximale oblongue (30) et une pièce partielle distale (20) dotée d'une boucle, dans lequel la boucle est réalisée en un matériau élastique flexible et est escamotable dans un ensemble oblong en forme de tube (40) entourant au moins partiellement le dispositif et peut être déployée hors de celui-ci, le plan de la boucle étant coudé d'un certain angle au moins dans sa configuration d'empreinte primaire par rapport à l'axe longitudinal de la pièce partielle (30) proximale oblongue,
**caractérisé en ce qu'**il est prévu
une boucle (21) au moins partiellement formée en double avec un centre de boucle, laquelle est modifiable par rapport à une configuration d'empreinte primaire quant à son centre de boucle et/ou sa forme et/ou son diamètre lors de l'escamotage dans ou du déploiement hors de l'ensemble (40).

2. Dispositif selon la revendication 1,
**caractérisé en ce que**
une région partielle en forme de V (22) est prévue, laquelle s'étend entre la boucle (21) et la pièce partielle proximale (30).

3. Dispositif selon la revendication 1 ou 2,
**caractérisé en ce que**
le diamètre de la boucle et/ou la forme de la boucle est modifiable sur commande par l'utilisation du dispositif (1) avec un ensemble (40) oblong en forme de tube entourant au moins partiellement le dispositif.

4. Dispositif selon la revendication 1, 2 ou 3,
**caractérisé en ce que**
le centre de la boucle (21) est positionné essentiellement à l'extérieur, notamment directement sur l'axe longitudinal de la pièce partielle (30) proximale oblongue.

5. Dispositif selon la revendication 1, 2, 3, ou 4,
**caractérisé en ce que**
la boucle (21) prend une forme essentiellement ronde ou ovale dans la configuration primaire et une forme de coeur dans une configuration secondaire.

6. Dispositif selon une des revendications précédentes,
**caractérisé en ce que**
la mise en forme de la boucle (21) est réglable de telle sorte que la boucle se centre d'elle-même à l'intérieur de la cavité corporelle ou du vaisseau et se conforme à la paroi du vaisseau ou de la cavité.

7. Dispositif selon une des revendications précédentes,
**caractérisé en ce que**
le plan de la boucle (21) est disposé selon un angle, en particulier essentiellement à angle droit par rapport à l'axe longitudinal de la pièce partielle (30) proximale oblongue et à la région partielle (22) en forme de V ou la boucle (21) est concave ou bombée en arc par rapport à l'axe longitudinal de la pièce partielle (30) proximale oblongue et à la région partielle (22) en forme de V et/ou par rapport à l'ensemble oblong (40) en forme de tube.

8. Dispositif selon une des revendications précédentes,
**caractérisé en ce que**
la boucle formée en double présente deux moitiés (28, 29) qui sont décalées l'une par rapport à l'autre dans des plans (E1, E2) qui se coupent.

9. Dispositif selon la revendication 8,
**caractérisé en ce que**
les deux moitiés sont assemblées en une seule pièce ou en deux parties, en particulier les deux moitiés présentent une jonction qui forme une saillie (23).

10. Dispositif selon la revendication 9,
**caractérisé en ce que**
la saillie (23) est en forme de noeud ou comprend un élément assemblé ou est formée par fusion de deux parties de boucle (28, 29).

11. Dispositif selon la revendication 10,
**caractérisé en ce que**
l'élément assemblé a la forme d'un fourreau ou est sphéroïdal, en particulier est une sphère atraumatique.

12. Dispositif selon une des revendications 9 à 11,
**caractérisé en ce que**
la saillie (23) en forme de noeud repose à un certain angle par rapport au ou aux plan(s) (E1, E2) de la boucle et/ou est disposée essentiellement parallèlement à l'axe longitudinal de la pièce partielle (30) proximale oblongue.

13. Dispositif selon une des revendications précédentes,
**caractérisé en ce que**
la pièce partielle distale présente une région partielle en forme de V (22) entre la boucle formée en double et la pièce partielle proximale oblongue.

14. Dispositif selon une des revendications précédentes,
**caractérisé en ce que**
les pièces partielles proximale et distale du dispositif sont formées en une seule pièce, la pièce partielle proximale (30) comprenant les extrémités du fil utilisé pour la boucle (21) et la région partielle (22) en forme de V.

15. Dispositif selon une des revendications précédentes,
**caractérisé en ce que**
la boucle présente un fil qui présente un ou plusieurs torons, en particulier le fil de boucle présente une ou plusieurs âme(s) de fil, entortillée(s) d'un ou plusieurs torons.

16. Dispositif selon une des revendications précédentes,
**caractérisé en ce que**
le fil présente une section essentiellement ronde, ovale, rectangulaire, polygonale, en forme d'étoile et/ou plate et le diamètre du fil est, sur la longueur du fil, essentiellement constant ou modifié de manière spécifique à chaque utilisation.

17. Dispositif selon une des revendications précédentes,
**caractérisé en ce que**
la boucle comprend un fil en matériau à mémoire de forme, en particulier en matériau superélastique.

18. Dispositif selon une des revendications précédentes,
**caractérisé en ce que**
la boucle est revêtue d'un métal noble et/ou présente un ou plusieurs fils ou un revêtement en un matériau visible par un appareil d'imagerie.
